(19) Europäisches Patentamt
European Patent Office
Office européen des brevets

(11) Veröffentlichungsnummer : **0 053 698**
**B1**

(12) # EUROPÄISCHE PATENTSCHRIFT

(45) Veröffentlichungstag der Patentschrift : **09.01.85**

(51) Int. Cl.⁴ : **C 07 D231/38**, A 01 N 43/56// C07C121/82, A01N37/34

(21) Anmeldenummer : **81108815.2**

(22) Anmeldetag : **23.10.81**

(54) **5-Amino-1-phenylpyrazol-4-carbonsäureester, Verfahren zu ihrer Herstellung, diese enthaltende Herbizide und ihre Anwendung als Herbizide.**

(30) Priorität : **05.12.80 DE 3045903**
**04.07.81 DE 3126479**

(43) Veröffentlichungstag der Anmeldung : **16.06.82 Patentblatt 82/24**

(45) Bekanntmachung des Hinweises auf die Patenterteilung : **09.01.85 Patentblatt 85/02**

(84) Benannte Vertragsstaaten : **AT BE CH DE FR GB IT LI LU NL SE**

(56) Entgegenhaltungen :
**EP-A- 0 002 180**
**EP-A- 0 034 945**
**GB-A- 854 632**
**US-A- 3 187 006**

(73) Patentinhaber : **BASF Aktiengesellschaft**
**Carl-Bosch-Strasse 38**
**D-6700 Ludwigshafen (DE)**

(72) Erfinder : **Eicken, Karl, Dr.**
**Waldstrasse 63**
**D-6706 Wachenheim (DE)**
Erfinder : **Plath, Peter, Dr.**
**Berner Weg 24**
**D-6700 Ludwigshafen (DE)**
Erfinder : **Wuerzer, Bruno, Dr. Dipl.-Landw.**
**Ruedigerstrasse 13**
**D-6701 Otterstadt (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 053 698 B1

**Beschreibung**

Die vorliegende Erfindung betrifft 5-Amino-1-phenylpyrazol-4-carbonsäureester, Verfahren zur Herstellung dieser Verbindungen, Herbizide, welche diese Verbindungen enthalten, sowie Verfahren zur Bekämpfung unerwünschten Pflanzenwuchses mit diesen Verbindungen.

5-Amino-1-phenylpyrazol-4-carbonsäureester, die ein Chloratom im Phenylrest besitzen, sind aus der Literatur bekannt (US-PS 3 567 735 ; Archiv Pharm. *312* (1979) 703 und EP-A-34945). Sie dienen als Zwischenprodukte z. B. für die Synthese von Diuretika, antibakteriellen Verbindungen oder Herbiziden. Über herbizide Eigenschaften dieser Verbindungen ist nichts bekannt.

Es ist ferner bekannt, 3-Anilino-4-carboxymethylpyrazole als Herbizide zu verwenden (EP-2180). Hierdurch werden die neuen Verbindungen nicht nahegelegt.

Es wurde nun gefunden, daß 5-Amino-1-phenylpyrazol-4-carbonsäureester der Formel I

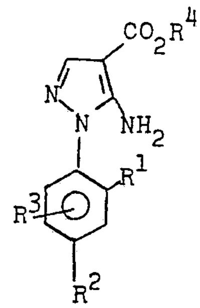

(I)

in der

$R^1$ Methyl, Trifluormethyl, Chlor oder Brom

$R^2$ Chlor, Brom, Jod oder $C_1$-$C_3$-Alkylsulfonyl,

$R^3$ Wasserstoff, Chlor oder Brom bedeutet oder Methoxi in 5-Stellung bedeutet, wobei dann $R^1$ und $R^2$ Chlor bedeuten,

oder $R^2$ Wasserstoff bedeutet, wenn $R^3$ Chlor in 3-Stellung bedeutet und

$R^4$ Alkyl mit 1 bis 3 Kohlenstoffatomen bedeutet, eine überraschend starke und gleichzeitig selektive Herbizide Wirkung aufweisen.

Die 5-Amino-1-phenylpyrazol-4-carbonsäurester der Formel I erhält man beispielsweise, indem man substituierte Phenylhydrazine der Formel II

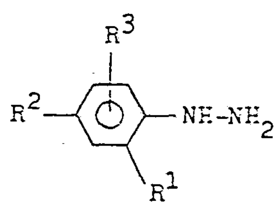

(II)

mit substituierten 2-Cyanacrylsäureestern der Formel III

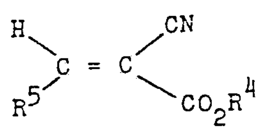

(III)

in der $R^5$ Alkoxi mit 1 bis 4 Kohlenstoffatomen oder N,N-Dialkylamino mit 1 bis zu 4 Kohlenstoffatomen je Alkylrest oder eine Hydroxigruppe bedeutet, gegebenenfalls in Gegenwart eines säurebindenden Mittels zu substituieren 2'-Phenylhydrazino-2-cyanacrylsäureestern der Formel IV

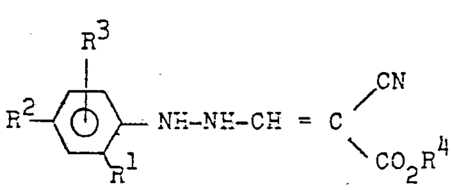

(IV)

bei Temperaturen unterhalb 70 °C umsetzt (erster Schritt) und diese Zwischenstufen in einem zweiten Schritt durch Erhitzen auf Temperaturen oberhalb 70 °C (Verfahren A) oder durch Behandeln mit wäßriger Mineralsäure bei Temperaturen zwischen 0° und 150 °C, vorzugsweise zwischen 20 °C und 100 °C cyclisiert (Verfahren B).

Als Lösungsmittel für das Verfahren A eignen sich insbesondere Alkohole, z. B. Methanol, Ethanol, es können jedoch auch Ether wie Dioxan, Tetrahydrofuran, Anisol oder Kohlenwasserstoffe wie Toluol, Xylol verwendet werden. Nach Ende der Reaktion beim ersten oder zweiten Schritt wird die Lösung gekühlt und das gebildete Produkt durch Abfiltrieren isoliert und gegebenenfalls durch Umkristallisieren gereinigt. Wird die Cyclisierung nach Verfahren B mit wäßriger Mineralsäure, vorzugsweise 5 bis 38 %iger (Gew.%) Salzsäure oder 5 bis 50 %iger Schwefelsäure, vorgenommen, so wird das Reaktionsgemisch nach Ende der Umsetzung mit dem doppelten bis zwanzigfachen Volumen Wasser verdünnt und das Cyclisierungsprodukt abgesaugt und unter Zugabe von verdünntem Alkali oder Ammoniak neutral gewaschen und gegebenenfalls umkristallisiert.

Ferner erhält man 5-Amino-1-phenylpyrazol-4-carbonsäureester der Formel I durch Umsetzung substituierter Phenylhydrazine der Formel II mit substituierten 2-Cyanacrylsäurestern der Formel III in einem Schritt bei Temperaturen oberhalb von 70 °C (Verfahren C). Als Lösungsmittel eignen sich die im Verfahren A benutzten Solventien, vorzugsweise Alkohole, deren Siedepunkte über 70 °C liegen. Die Isolierung der Endprodukte erfolgt in der im Verfahren A beschriebenen Weise. Die 2-Cyanacrylsäureester der Formel III werden in mindestens molarer Menge, bezogen auf die substituierten Phenylhydrazine der Formel II eingesetzt, vorzugsweise in stöchiometrischer Menge. Werden im Verfahren A oder C anstelle der freien Phenylhydrazine der Formel II, deren mineralsaure Salze, wie z. B. Hydrochloride oder Sulfate eingesetzt, so ist es zweckmäßig zuerst das substituierte Phenylhydrazin der Formel II durch Zugabe einer äquivalenten Menge an Alkalialkoholaten oder Natriumacetat freizusetzen und dann die Umsetzung auszuführen.

Die verwendeten Phenylhydrazine der Formel II sind bekannt oder können nach bekannten Methoden hergestellt werden. (Methoden der Organ. Chemie, Houben Weyl, Bd. 10/2, S. 180 ff). Die eingesetzten 2-Cyanacrylsäureester der Formel III sind bekannt. (DOS 2 635 841 ; Chem. Ber. (1964) *97,* 3397).

Die bei der Durchführung des Verfahrens A isolierten 2'-Phenylhydrazin-2-cyanacrylsäurester der Formel IV besitzen ebenfalls herbizide Eigenschaften. Die folgenden Beispiele erläutern die Herstellung der Zwischenprodukte und der Endprodukte.

In den Beispielen verhalten sich Gewichststeile zu Volumenteilen wie Kilogramm zu Liter.

Herstellung der 2'-Phenylhydrazino-2-cyanacrylsäureester der Formel IV :

## Beispiel A

148,1 Gewichtsteile 2,4,6-Trichlorphenylhydrazin werden in eine Lösung von 108,5 Gewichtsteilen Ethoximethylen-2-cyanessigsäuremethylester in 1 000 Volumenteilen Methanol gegeben. Aus der Lösung fällt ein Kristallbrei aus, der nach 3-stündigem Rühren, Absaugen und Trocknen i. Vac. bei 40 °C 187,4 Gewichtsteile 2'-(2,4,6-Trichlorphenyl)-hydrazino-2-cyanacrylsäuremethylester vom Fp. : 174 bis 175 °C liefert.

$C_{11}H_8Cl_3N_3O_2$ (M 320,5)

| | | | |
|---|---|---|---|
| ber. : | C 41,22 | H 2,52 | N 13,11 |
| gef. : | C 40,9 | H 2,8 | N 12,8 |

## Beispiel B

Eine Suspension von 21,4 Gewichtsteilen 2,4-Dichlorphenyl-hydrazin-Hydrochlorid in 150 Volumenteilen Methanol wird durch Zusatz von ca. 18 Gewichtsteilen 30 %iger Natriumethylatlösung neutralisiert und nach Zugabe von 15,5 Gewichtsteilen Ethoximethylen-2-cyanessigsäuremethylester 3 Stunden bei 25 °C gerührt, durch 15 Minuten am Rückfluß erhitzt. Aus dem Filtrat isoliert man nach Verdampfen des Methanols i. Vac. und Umkristallisieren aus Ethanol (bei 50 °C) 17,5 Gewichtsteile 2'-(2,4-Dichlorphenyl)-hydrazino-2-cyanacrylsäuremethylester vom Fp. : 154 bis 156 °C.

$C_{11}H_9Cl_2N_3O_2$ (M 286)

| | | | |
|---|---|---|---|
| ber. : | C 46,18 | H 3,17 | N 14,69 |
| gef. : | C 46,0 | H 3,2 | N 14,8 |

In entsprechender Weise können folgende 2'-Phenylhydrazino-2-cyanacrylsäureester der Formel IV hergestellt werden :

(Siehe Tabelle Seite 4 f.)

Tabelle

| $R^1$ | $R^2$ | $R^3$ | $R^4$ | Fp:(°C) |
|---|---|---|---|---|
| $CH_3$ | Cl | H | $CH_3$ | 140 |
| Br | Br | 6-Br | $CH_3$ | 182 |
| Cl | Br | 5-Cl | $CH_3$ | |
| Cl | Cl | 5-Cl | $CH_3$ | 195 |
| $CH_3$ | Br | H | $CH_3$ | |
| $CH_3$ | Br | 6-Br | $CH_3$ | |
| $CH_3$ | Cl | 6-Cl | $CH_3$ | |
| Cl | Cl | 6-Br | $CH_3$ | |
| Cl | Br | 6-Br | $CH_3$ | |
| Cl | Br | 6-Cl | $CH_3$ | |
| Br | Cl | 6-Br | $CH_3$ | |
| Br | Br | H | $CH_3$ | |
| Cl | Cl | 5-$CH_3$O | $CH_3$ | |
| Cl | Cl | H | $C_2H_5$ | 175 |
| Cl | Cl | 6-Cl | $C_2H_5$ | 166 |
| Cl | Cl | 6-Cl | i-$C_3H_7$ | 130 |

Beispiel 1 (Verfahren B)

90,0 Gewichtsteile 2'-(2,4,6-Trichlorphenyl)-hydrazino-2-cyanacrylsäuremethylester werden in 300 Volumenteilen 18 %iger Salzsäure 5 Stdn. bei 80 °C gerührt. Nach dem Abkühlen und Verdünnen mit 500 Volumenteilen Wasser isoliert man nach dem Absaugen und neutral waschen mit Wasser und Natriumbicarbonatlösung 70,6 Gewichtsteile 5-Amino-1-(2,4,6-trichlorphenyl)-pyrazol-4-carbonsäuremethylester vom Fp.: 179 bis 180 °C (Wirkstoff Nr. 1).

$C_{11}H_8Cl_3N_3O_2$ (M 320,5)
ber.: C 41,22 H 2,52 N 13,11
gef.: C 41,4 H 2,8 N 12,6

Beispiel 2 (Verfahren B)

15,0 Gewichtsteile 2'-(2,4-Dichlorphenyl)-hydrazino-2-cyanacrylsäuremethylester werden in 50 Volumenteilen konz. Salzsäure 12 Stdn. bei 25° gerührt und danach in 500 Volumenteile Eiswasser eingerührt. Der ausgefallene Niederschlag ergibt nach Absaugen und neutral waschen mit Wasser und Natriumbicarbonatlösung 14,2 Gewichtsteile 5-Amino-1-(2,4-dichlorphenyl)-pyrazol-4-carbonsäuremethylester vom Fp. 143 bis 145 °C (Wirkstoff Nr. 2).

$C_{11}H_9Cl_2N_3O_2$ (M 286)
ber.: C 46,18 H 3,17 N 14,69
gef.: C 46,2 H 3,2 N 14,7

Beispiel 3 (Verfahren A)

45,0 Gewichtsteile 2'-(2,4-Dichlorphenyl)-hydrazino-2-cyanacrylsäuremethylester werden in 200 Volumenteilen n-Propanol 6 Stunden am Rückfluß erhitzt. Nach dem Abkühlen isoliert man durch Absaugen 34,6 Gewichtsteile 5-Amino-1-(2,4-dichlorphenyl)-pyrazol-4-carbonsäuremethylester vom Fp.: 144 bis 145 °C (Wirkstoff Nr. 2).

Beispiel 4 (Verfahren C)

21,2 Gewichtsteile 2,4,6-Trichlorphenylhydrazin und 15,5 Gewichtsteile Ethoximethylen-2-cyanessigsäuremethylester werden in 120 Volumenteilen n-Butanol 2 Stdn. am Rückfluß erhitzt. Nach dem Abkühlen isoliert man durch Absaugen 22,8 Gewichtsteile 5-Amino-1-(2,4,6-trichlorphenyl)-pyrazol-4-carbonsäuremethylester vom Fp.: 180 bis 181 °C (Wirkstoff Nr. 1).

Tabelle

| Nr. | $R^1$ | $R^2$ | $R^3$ | $R^4$ | Fp (°C) |
|---|---|---|---|---|---|
| 3 | Cl | Cl | H | $C_2H_5$ | 109 |
| 4 | Cl | Cl | 6-Cl | $C_2H_5$ | 105 |
| 5 | Cl | Cl | 6-Cl | i-$C_3H_7$ | 152 |
| 6 | $CH_3$ | Cl | H | $CH_3$ | 109 |
| 7 | Br | Br | 6-Br | $CH_3$ | 194 |
| 8 | Cl | Br | 5-Cl | $CH_3$ | 173 |
| 9 | Cl | Cl | 5-Cl | $CH_3$ | 175 |
| 10 | $CH_3$ | Br | H | $CH_3$ | 123 |
| 11 | $CH_3$ | Br | 6-Br | $CH_3$ | 175 |
| 12 | $CH_3$ | Cl | 6-Cl | $CH_3$ | |
| 13 | Cl | Cl | 6-Br | $CH_3$ | 199 |
| 14 | Cl | Br | 6-Br | $CH_3$ | 208 |
| 15 | Cl | Br | 6-Cl | $CH_3$ | |
| 16 | Br | Cl | 6-Br | $CH_3$ | 172 |
| 17 | Br | Br | H | $CH_3$ | |
| 18 | Cl | Cl | 5-$CH_3$O | $CH_3$ | 182 |
| 19 | Cl | Br | 6-Cl | $CH_3$ | |
| 20 | Cl | J | H | $CH_3$ | 180 |
| 21 | Cl | H | 3-Cl | $CH_3$ | 164 |
| 22 | Cl | Cl | 6-Cl | n-$C_3H_7$ | |
| 23 | $CF_3$ | Cl | H | $CH_3$ | 146 |
| 24 | Cl | $-SO_2CH_3$ | H | $CH_3$ | 215 |

Die Anwendung als Herbizide erfolgt z. B. in Form von direkt versprühbaren Lösungen, Pulvern, Suspensionen oder Dispersionen, Emulsionen, Öldispersionen, Pasten, Stäubemitteln, Streumitteln, Granulaten durch Versprüchen, Vernebeln, Verstäuben, Verstreuen oder Gießen. Die Anwendungsformen richten sich ganz nach den Verwendungszwecken ; sie sollen in jedem Fall möglichst die feinste Verteilung der neuen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische cyclische und aromatische Kohlenwasserstoffe, zum Beispiel Benzol, Toluol, Xylol, Paraffin, Tetrahydronaphthalin, alkylierte Naphthaline oder deren Derivate zum Beispiel Methanol, Äthanol, Propanol, Butanol, Chloroform, Tetrachlorkohlenstoff, Cyclohexanol, Cyclohexanon, Chlorbenzol, Isophoron usw., stark polare Lösungsmittel, z. B. Dimethylformamid, Dimethylsulfoxid, N-Methylpyrrolidon, Wasser usw. in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Die Herbizide enthalten z. B. 5 bis 95 % (Gew.%) insbesondere 10 bis 80 % Wirkstoff.

An oberflächenaktiven Stoffen sind zu nennen : Alkali-, Erdalkali-, Ammoniumsalze von Ligninsulfonsäure, Naphthalinsulfonsäuren, Phenolsulfonsäuren, Alkylarylsulfonate, Alkylsulfate, Alkylsulfonate, Alkali- und Erdalkalisalze der Dibutylnaphthalinsulfonsäure, Lauryläthersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykoläther, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylen-octylphenolether, äthoxyliertes

Isooctylphenol-, Octylphenol-, Nonylphenol, Alkylphenolpolyglykoläther, Tributylphenylpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden wie Kieselsäuren, Silikate, Talkum, Kaolin, Kalk, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehle, Baumringen-, Holz- und Nußschalenmehl, Cellulosepulver und andere feste Trägerstoffe.

Beispiel a

Man vermischt 90 Gewichtsteile der Verbindung 1 mit 10 Gewichtsteilen N-Methyl-alpha-pyrrolidon und erhält eine Lösung, die zur Anwendung in Form kleinster Tropfen geeignet ist.

Beispiel b

10 Gewichtsteile der Verbindung 1 werden in einer Mischung gelöst, die aus 90 Gewichtsteilen Xylol, 6 Gewichtsteilen des Anlagerungsproduktes von 8 bis 10 Mol Ethylenoxid an 1 Mol Ölsäure-N-monoethanolamid, 2 Gewichtsteilen Calciumsalz der Dodecylbenzolsulfonsäure und 2 Gewichtsteilen des Anlagerungsproduktes von 40 Mol Ethylenoxid an 1 Mol Rizinusöl besteht.

Beispiel c

20 Gewichtsteile der Verbindung 1 werden in einer Mischung gelöst, die aus 60 Gewichtsteilen Cyclohexanon, 30 Gewichtsteilen Isobutanol, 5 Gewichtsteilen des Anlagerungsproduktes von 7 Mol Ethylenoxid an 1 Mol Isooctylphenol und 5 Gewichtsteilen des Anlagerungsproduktes von 40 Mol Ethylenoxid an 1 Mol Rizinusöl besteht.

Beispiel d

20 Gewichtsteile der Verbindung 1 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Rizinusöl besteht.

Beispiel e

80 Gewichtsteile des Wirkstoffs 1 werden mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfit-Ablauge und 7 Gewichtsteilen pulverfömigem Kieselsäuregel gut vermischt und in einer Hammermühle vermahlen.

Beispiel f

5 Gewichsteile der Verbindung 1 werden mit 95 Gewichtsteielen feinteiligem Kaolin innig vermischt. Man erhält auf diese Weise ein Stäubemittel, das 5 Gewichtsprozent des Wirkstoffs enthält.

Beispiel g

30 Gewichtsprozent der Verbindung 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Beispiel h

40 Gewichtsteile des Wirkstoffs 1 werden mit 10 Teilen Natriumsalz eines Phenolsulfonsäure-harnstoff-formaldehyd-kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gewichtsprozent Wirkstoff enthält.

Beispiel i

20 Teile des Wirkstoffs 1 werden mit 12 Teilen Calciumsalz der Dodecylbenzolsulfonsäure, 8 Teile Fettalkohol-polyglykolether, 2 Teilen Natriumsalz eines Phenolsulfonsäure-harnstoff-formaldehyd-kondensats und 68 Teilen eines paraffinischen Mineralöls innig vermischt. Man erhält eine stabile ölige Dispersion.

Der Einfluß von Vertretern der neuen 5-Amino-1-phenylpyrazol-4-carbonsäureester auf das Wachstum von erwünschten und unerwünschten Pflanzen wird anhand von Gewächshausversuchen vorgeführt :

Als Kulturgefäße dienten Plastikblumentöpfe mit 300 $cm^3$ Inhalt und lehmiger Sand mit etwa 1,5 % Humus als Substrat. Die Samen der Testpflanzen wurden nach Arten getrennt flach eingesät. Unmittelbar danach erfolgte bei Vorauflaufbehandlung das Aufbringen der Wirkstoffe auf die Erdoberfläche. Sie wurden hierbei in Wasser als Verteilungsmittel suspendiert oder emulgiert und mittels fein verteilender Düsen gespritzt. Bei deser Applikationsmethode handelt es sich um eine Aufwandmenge entsprechend 3,0 kg Wirkstoff/ha. Nach dem Aufbringen der Mittel wurden die Gefäße leicht beregnet, um Keimung und Wachstum in Gang zu bringen. Danach deckte man die Gefäße mit durchsichtigen Plastikhauben abe, bis die Pflanzen angewachsen waren. Diese Abdeckung bewirkte ein gleichmäßiges Keimen der Testpflanzen, sofern dies nicht durch die Wirkstoffe beeinträchtig wurden. Zum Zwecke der Nachauflaufbehandlung zog man die Testpflanzen je nach Wuchsform erst bis zu einer Wuchshöhe von 3 bis 10 cm an und behandelte sie danach.

Zur Nachauflaufbehandlung wurden entweder direkt gesäte und in den gleichen Gefäßen aufgewachsene Pflanzen ausgewählt, oder sie wurden erst als Keimpflanzen getrennt angezogen und einige Tage vor der Behandlung in die Versuchsgefäße verpflanzt. Es ist zu erwähnen, daß bei dem für das Nachauflaufverfahren benutzten Reis das Substrat mit Torfmull (peat) angereichert war. Dasselbe gilt für Klettenlabkraut. Die Aufwandmenge für die Nachauflaufbehandlung betrug 0,5 kg/ha Wirkstoff für die neuen Verbindungen Nr. 1, 2, 6, 7.

Vertreter der zu Herstellung der 5-Amino-1-phenylpyrazol-4-carbonsäureester verwendeten 2'-Phenylhydrazino-2-cyanacrylsäureester (Beispiel A, Beispiel B) mit herbiziden Eigenschaften wurden mit 3,0 kg Wirkstoff je ha ebenfalls im Vor- und im Nachauflaufverfahren eingesetzt.

Allgemein unterblieb bei der Nachauflaufbehandlung die Abdeckung. Die Aufstellung der Versuche erfolgte im Gewächshaus, wobei für wärmeliebende Arten wärmere Bereiche (20 bis 35 °C) und für solche gemäßigter Klimate 15 bis 25 °C bevorzugt wurden. Die Versuchsperiode erstreckte sich über 2 bis 3 Wochen. Während dieser Zeit wurden die Pflanzen gepflegt und ihre Reaktion auf die einzelnen Behandlungen ausgewertet. Bewertet wurde nach einer Skala von 0 bis 100. Dabei bedeutet 0 keine Schädigung oder normaler Auflauf und 100 kein Aufgang der Pflanzen bzw. völlige Zerstörung zumindest der oberirdischen Teile.

Die Ergebnisse zeigen, daß die Verbindungen Nr. 1, 2, 3, 4, 6 und 7 im Gewächshaus bei Vorauflaufanwendung und einer Aufwandmenge von 3 kg Wirkstoff je ha eine beachtliche herbizide Wirkung besitzen.

Die Gewächshausversuche ergeben ferner, daß die Wirkstoffe gemäß Beispiel A und Beispiel B mit 3,0 kg Wirkstoff/ha bei Vor- und Nachauflaufanwendung eine gute herbizide Wirkung haben.

In diesen Gewächshausversuchen haben die Verbindungen Nr. 1, 2, 6 und 7 mit 0,5 kg Wirkstoff/ha eine sehr gute und breite Wirkung gegen zahlreiche breitblättrige unerwünschte Pflanzen. Dabei werden Getreidearten wie Winterweizen weitgehend geschont oder nur vorübergehend geringfügig geschädigt.

Als weiteres Resultat dieser Gewächshausversuche zeigte sich Verbindung Nr. 8 mit 1,0 kg Wirkstoff/ha im Nachauflaufverfahren ausgebracht als herbizid gut wirksam gegen einige breitblättrige Unkräuter und ohne, oder nur mit geringen Schäden verträglich für einige Kulturpflanzen.

Die als Vergleichsverbindung herangezogene bekannte Verbindung A hatte mit 2,0 kg Wirkstoff/ha im Nachauflaufverfahren angewandt keine nennenswerte herbizide Aktivität.

Weiterhin erbrachten die beschriebenen Gewächshausversuche bei Nachauflaufanwendung von 0,5 kg Wirkstoff/ha der Verbindung Nr. 13 eine sehr gute herbizide Wirkung gegen breitblättrige Unkräuter, ohne die Getreidearten zu schädigen. Desgleichen bekämpfte die Verbindung Nr. 16 in denselben Versuchen mit 1,0 kg Wirkstoff/ha breitblättrige Unkräuter bei nur geringen und temporären Schädigungen des Getreides.

Sind Kulturpflanzen bei Blattbehandlung gegenüber den Wirkstoffen etwas empfindlich, so können auch Ausbringungstechniken angewandt werden, bei welchen die herbiziden Mittel mit Hilfe der Spritzgeräte so geleitet werden, daß die Blätter empfindlicher Kulturpflanzen nach Möglichkeit nicht getroffen werden, während sie auf die Blätter darunter wachsender unerwünschter Pflanzen oder die unbedeckte Bondenfläche gelangen (post direkcted, lay-by). In Anbetracht der Vielseitigkeit der Applikationsmethode können die erfindungsgemäßen Herbizide noch in einer weiteren großen Zahl von Kulturpflanzen zur Beseitigung unerwünschten Pflanzenwuchses eingesetzt werden. Die Aufwandmengen können dabei zwischen 0,1 und 15 kg/ha und mehr schwanken.

In Betracht kommen beispielsweise die folgenden Kulturen :

(Siehe Tabelle Seite 8 f.)

Tabelle

| Botanischer Name | Deutscher Name | Englischer Name |
|---|---|---|
| Allium cepa | Küchenzwiebel | onions |
| Ananas comosus | Ananas | pineapple |
| Arachis hypogaea | Erdnuß | peanuts (groundnuts) |
| Asparagus officinalis | Spargel | asparagus |
| Avena sativa | Hafer | oats |
| Beta vulgaris spp. altissima | Zuckerrübe | sugarbeets |
| Beta vulgaris spp. rapa | Futterrübe | fooder beets |
| Beta vulgaris spp. esculenta | Rote Rübe | table beets, red beets |
| Brassica napus var. napus | Raps | rape seed |
| Brassica napus var. napobrassica | Kohlrübe | |
| Brassica napus var. rapa | Weiße Rübe | turnips |
| Brassica rapa var. silvestris | Rübsen | |
| Camellia sinensis | Teestrauch | tea plants |
| Carthamus tinctorius | Saflor - Färberdistel | safflower |
| Carya illinoinensis | Pekannußbaum | pecan trees |
| Citrus limon | Zitrone | lemon |
| Citrus maxima | Pampelmuse | grapefruits |
| Citrus reticulata | Mandarine | |
| Citrus sinensis | Apfelsine, Orange | orange trees |
| Coffea arabica (Coffea canephora, Coffea liberica) | Kaffee | coffee plants |
| Cucumis melo | Melone | melons |

(Fortsetzung)

| Botanischer Name | Deutscher Name | Englischer Name |
|---|---|---|
| Cucumis sativus | Gurke | cucumber |
| Cynodon dactylon | Bermudagras | Bermudagrass in turfs and lawn |
| Daucus carota | Möhre | carrots |
| Elaeis guineensis | Ölpalme | oil palms |
| Fragaria vesca | Erdbeere | strawberries |
| Glyoine max | Sojabohne | soybeans |
| Gossypium hirsutum (Gossypium arboreum Gossypium herbaceum Gossypium vitifolium) | Baumwolle | cotton |
| Helianthus annuus | Sonnenblume | sunflowers |
| Helianthus tuberosus | Topinambur | |
| Hevea brasiliensis | Parakatuschukbaum | rubber plants |
| Hordeum vulgare | Gerste | barley |
| Humulus lupulus | Hopfen | hop |
| Ipomoea batatas | Süßkartoffeln | sweet potato |
| Juglans regia | Walnußbaum | walnut trees |
| Lactua sativa | Kopfsalat | lettuce |
| Lens culinaris | Linse | lentils |
| Linum usitatissimum | Faserlein | flax |
| Lycopersicon lycopersicum | Tomate | tomato |
| Malus spp. | Apfel | apple trees |

(Fortsetzung)

| Botanischer Name | Deutscher Name | Englischer Name |
|---|---|---|
| Manihot esculenta | Maniok | cassava |
| Medicago sativa | Luzerne | alfalfa (lucerne) |
| Metha piperita | Pfefferminze | peppermint |
| Musa spp. | Obst- und Mehlbanane | banana plants |
| Nicotiana tabacum (N. rustica) | Tabak | tabacco |
| Olea europaea | Ölbaum | olive trees |
| Oryza sativa | Reis | rice |
| Panicum miliaceum | Rispenhirse | |
| Phaseolus lunatus | Mondbohne | limabeans |
| Phaseolus mungo | Urdbohne | mungbeans |
| Phaseolus vulgaris | Buschbohnen | snapbeans, green beans, dry beans |
| Pennisetum glaucum | Perl- oder Rohrkolben-hirse | |
| Petroselinum crispum spp. tuberosum | Wurzelpetersilie | parsley |
| Picea abies | Rotfichte | Norway spruce |
| Abies alba | Weißtanne | fire |
| Pinus spp. | Kiefer | pine trees |
| Pisum sativum | Gartenerbse | English peas |
| Prunus avium | Süßkirsche | cherry trees |
| Prunus domestica | Pflaume | plum trees |

(Fortsetzung)

| Botanischer Name | Deutscher Name | Englischer Name |
|---|---|---|
| Prunus dulcis | Mandelbaum | almond trees |
| Prunus persica | Pfirsich | peach trees |
| Pyrus communis | Birne | pear trees |
| Ribes sylvestre | Rote Johannisbeere | red currants |
| Ribes uva-crispa | Stachelbeere | |
| Ricinus communis | Rizinus | |
| Saccharum officinarum | Zuckerrohr | sugar cane |
| Secale cereale | Roggen | rye |
| Sasamum indicum | Sesam | Sesame |
| Solanum tuberosum | Kartoffel | Irish potatoes |
| Sorghum bicolor (s. vulgare) | Mohrenhirse | sorghum |
| Sorghum dochna | Zuckerhirse | |
| Spinacia oleracea | Spinat | spinach |
| Theobroma cacao | Kakaobaum | cacao plants |
| Trifolium pratense | Rotklee | red clover |
| Triticum aestivum | Weizen | wheat |
| Vaccinium corymbosum | Kulturheidelbeere | blueberry |
| Vaccinium vitis-idaea | Preißelbeere | cranberry |
| Vicia faba | Pferdebohnen | tick beans |
| Vigna sinensis (V. unguiculata) | Kuhbohne | cow peas |
| Vitis vinifera | Weinrebe | grapes |
| Zea mays | Mais | Indian corn, sweet corn maize |

Zur Verbreiterung des Wirkungsspektrums und nur Erzielung synergistischer Effekte können die neuen 5-Amino-1-phenyl-pyrazol-4-carbonsäureester mit zahlreichen Vertretern anderer herbizider oder wachstumsreglierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäure, Triazine, Amide, Harnstoffe, Diphenylether, Triazinone, Uracile, Benzofuranderivate, Cyclohexan-1,3-dion-derivate und andere in Betracht. Eine Reihe von Wirkstoffen, welche zusammen mit den neuen Verbindungen für verschiedenste Anwendungsbereiche sinnvolle Mischungen ergeben, werden beispielshaft aufgeführt:

5-Amino-4-chlor-2-phenyl-3(2H)-pyridazinon
5-Amino-4-brom-2-phenyl-3(2H)-pyridazinon
5-Amino-4-chlor-2-cyclohexyl-3(2H)-pyridazinon
5-Amino-4-brom-2-cyclohexyl-3(2H)-pyridazinon

5-Methylamino-4-chlor-2-(3-trifluormethylphenyl)-3(2H)-pyridazinon
5-Methylamino-4-chlor-2-(3- „β,β-tetrafluorethoxyphenyl)-3(2H)-pyridazinon
5-Dimethylamino-4-chlor-2-phenyl-3(2H)-pyridazinon
4,5-Dimethoxy-2-phenyl-3(2H)-pyridazinon
4,5-Dimethoxy-2-cyclohexyl-3(2H)-pyridazinon
4,5-Dimethoxy-2-(3-trifluormethylphenyl)-3(2H)-pyridazinon
5-Methoxy-4-chlor-2-(3-trifluormethylphenyl)-3(2H)-pyridazinon
5-Amino-4-brom-2-(3-methylphenyl)-3(2H)-pyridazinon

3-(1-Methylethyl)-1H-2,1,3-benzothiadiazin-4(3H)-on-2,2-dioxid und Salze
3-(1-Methylethyl)-8-chlor-1H-2,1,3-benzothiadiazin-4(3H)-on-2,2-dioxid und Salze
3-(1-Methylethyl)-8-fluor-1H-2,1,3-benzothiadiazin-4(3H)-on-2,2-dioxid und Salze
3-(1-Methylethyl)-8-methyl-1H-2,1,3-benzothiadiazin-4(3H)-on-2,2-dioxid und Salze

1-Methoxymethyl-3-(1-methylethyl)-2,1,3-benzothiadiazin-4(3H)-on-2,2-dioxid
1-Methoxymethyl-8-chlor-3-(1-methylethyl)-2,1,3-benzothiadiazin-4(3H)-on-2,2-dioxid
1-Methoxymethyl-8-fluor-3-(1-methylethyl)-2,1,3-benzothiadiazin-4(3H)-on-2,2-dioxid
1-Cyan-8-chlor-3-(1-methylethyl)-2,1,3-benzothiadiazin-4(3H)-on-2,2-dioxid
1-Cyan-8-fluor-3-(1-methylethyl)-2,1,3-benzothiadiazin-4(3H)-on-2,2-dioxid
1-Cyan-8-methyl-3-(1-methylethyl)-2,1,3-benzothiadiazin-4(3H)-on-2,2-dioxid
1-Cyan-3-(1-methylethyl)-2,1,3-benzothiadiazin-4(3H)-on-2,2-dioxid
1-Azidomethyl-3-(1-methylethyl)-2,1,3-benzothiadiazin-4(3H)-on-2,2-dioxid
3-(1-Methylethyl)-1H-pyridino-[3,2-e]2,1,3-thiadiazin-(4)-on-2,2-dioxid

N-(1-Ethylpropyl)-2,6-dinitro-3,4-dimethylanilin
N-(1-Methylethyl)-N-ethyl-2,6-dinitro-4-trifluormethyl-anilin
N-n-Propyl-N-β-chlorethyl-2,6-dinitro-4-trifluormethyl-anilin
N-n-Propyl-N-cyclopropylmethyl-2,6-dinitro-4-trifluormethyl-anilin
N-Bis-(n-propyl)-2,6-dinitro-3-amino-4-trifluormethylanilin
N-Bis-(n-propyl)-2,6-dinitro-4-methyl-anilin
N-Bis-(n-propyl)-2,6-dinitro-4-methylsulfonyl-anilin
N-Bis-(n-propyl)-2,6-dinitro-4-aminosulfonyl-anilin
Bis-(β-chlorethyl)-2,6-dinitro-4-methyl-anilin
N-Ethyl-N-(2-methylallyl)-2,6-dinitro-4-trifluormethyl-anilin

N-Methylcarbaminsäure-3,4-dichlorbenzylester
N-Methylcarbaminsäure-2,6-di-tert-butyl-4-methylphenylester
N-Phenylcarbaminsäure-isopropylester
N-3-Fluorphenylcarbaminsäure-3-methoxypropyl-2-ester
N-3-Chlorphenylcarbaminsäure-isopropylester
N-3-Chlorphenylcarbaminsäure-butin-1-yl-3-ester
N-3-Chlorphenylcarbaminsäure-4-chlor-butin-2-yl-1-ester
N-3,4-Dichlorphenylcarbaminsäure-methylester
N-(4-Amino-benzolsulfonyl)-carbaminsäure-methylester
O-(N-Phenylcarbamoyl)-propanonoxim
N-Ethyl-2-(phenylcarbamoyl)-oxypropionsäureamid
3'-N-Isopropyl-carbamoyloxy-propionsäureanilid
Ethyl-N-(3'-phenylcarbamoyloxy)-phenyl)-carbamat
Methyl-N-(3-(N'-methyl-N'-phenylcarbamoyloxy)-phenyl)-carbamat
Isopropyl-N-(3-(N'-ethyl-N'-phenylcarbamoyloxy)-phenyl)-carbamat
Methyl-N-(3-N'-3-methylphenylcarbamoyloxy)-phenyl)-carbamat

Methyl-N-(3-N'-4-fluorphenylcarbamoyloxy)-phenyl)-carbamat
Methyl-N-(3-N'-3-chlor-4-fluorphenylcarbamoyloxy)-phenyl)-carbamat
Methyl-N-dichlorfluormethylsulfenyl-(3-(N'-dichlorfluormethylsulfenyl-N'-phenylcarbamoyl-oxy)-phenyl)-carbamat
Methyl-N-dichlorfluormethylsulfenyl-3-(N'-dichlorfluormethylsulfenyl-N'-3-methylphenylcarbamoyl-oxy)-phenyl)-carbamat

Ethyl-N-(3-N'-3-chlor-4-fluorphenylcarbamoyloxy)-phenyl)-carbamat
Ethyl-N-(3-N'-3,4-difluorphenylcarbamoyloxy)-phenyl)-carbamat
Methyl-N-(3-(N'-3,4-difluorphenylcarbamoyloxy)-phenyl)-carbamat
N-3-(4-Fluorphenoxycarbonylamino)-phenyl-carbaminsäure-methylester
N-3-(2-Methylphenoxycarbonylamino)-phenyl-carbaminsäure-ethylester
N-3-(4-Fluorphenoxycarbonylamino)-phenyl-thiolcarbaminsäuremethylester
N-3-(2,4,5-Trimethylphenoxycarbonylamino)-phenyl-thiolcarbaminsäure-methylester
N-3-(Phenoxycarbonylamino)-phenyl-thiolcarbaminsäure-methylester
N,N-Diethyl-thiolcarbaminsäure-p-chlorbenzylester
N,N-Di-n-propyl-thiolcarbaminsäure-ethylester
N,N-Di-n-propyl-thiolcarbaminsäure-n-propylester
N,N-Di-isopropyl-thiolcarbaminsäure-2,3-dichlorallylester
N,N-Di-isopropyl-thiolcarbaminsäure-2,3,3-trichlorallylester
N,N-Di-isopropyl-thiolcarbaminsäure-3-methyl-5-isoxazolylmethylester
N,N-Di-isopropyl-thiolcarbaminsäure-3-ethyl-5-isoxazolylmethylester
N,N-Di-sec.-butyl-thiolcarbaminsäure-ethylester
N,N-Di-sec.-butyl-thiolcarbaminsäure-benzylester
N-Ethyl-N-cyclohexyl-thiolcarbaminsäure-ethylester
N-Ethyl-N-bicyclo[2.2.1]heptyl-thiolcarbaminsäureethylester

S-(2,3-Dichlorallyl)-(2,2,4-trimethyl-azetidin)-1-carbothiolat
S-(2,3,3-Trichlorallyl)-(2,2,4-trimethyl-azetidin)-1-carbothiolat
S-Ethyl-hexahydro-1-H-azepin-1-carbothiolat
S-Benzyl-(3-methyl-hexahydro-1-H-azepin-1)-carbothiolat
S-Benzyl-(2,3-dimethylhexahydro-1-H-azepin-1)-carbothiolat
S-Ethyl-(3-methylhexahydro-1-H-azepin-1)-carbothiolat
N-Ethyl-N-n-butyl-thiolcarbaminsäure-n-propylester
N,N-Dimethyl-dithiocarbaminsäure-2-chlorallylester
N-Methyl-dithiocarbaminsäure-Natriumsalz
Trichloressigsäure-Natriumsalz
α,α-Dichlorpropionsäure-Natriumsalz
α,α-Dichlorbuttersäure-Natriumsalz
α,α,β,β-Tetrafluorpropionsäure-Natriumsalz
α-Methyl-α,β-dichlorpropionsäure-Natriumsalz
α-Chlor-β-(4-chlorphenyl)-propionsäure-methylester
α,β-Dichlor-β-phenylpropionsäure-methylester
Benzamido-oxy-essigsäure

2,3,5-Trijodbenzoesäure (Salze, Ester, Amide)
2,3,6-Trichlorbenzoesäure (Salze, Ester, Amide)
2,3,5,6-Tetrachlorbenzoesäure (Salze, Ester, Amide)
2-Methoxy-3,6-dichlorbenzoesäure (Salze, Ester, Amide)
2-Methoxy-3,5,6-trichlorbenzoesäure (Salze, Ester, Amide)
3-Amino-2,5,6-trichlorbenzoesäure (Salze, Ester, Amide)
O,S-Dimethyl-tetrachlor-thioterephthalat
Dimethyl-2,3,5,6-tetrachlor-terephthalat
Di-natrium-3,6-endoxohexahydro-phthalat
4-Amino-3,5,6-trichlor-picolinsäure (Salze)
2-Cyan-3-(N-methyl-N-phenyl)-amino-acrylsäureethylester
2-[4-(4'-Chlorphenoxy)-phenoxy]-propionsäureisobutylester
2-[4-(2',4'-Dichlorphenoxy)-phenoxy]-propionsäuremethylester
2-[4-(4'-Trifluormethylphenoxy)-phenoxy]-propionsäuremethylester
2-[4-(2'-Chlor-4'-trifluorphenoxy)-phenoxy]-propionsäure-Natriumsalz
2-[4-(3',5,-Dichlorpyridyl-2-oxy)-phenoxy]-propionsäure-Natriumsalz

2-(N-Benzoyl-3,4-dichlorphenylamino)-propionsäureethylester
2-(N-Benzoyl-3-chlor-4-fluorphenylamino)-propionsäure-methylester
2-(N-Benzoyl-3-chlor-4-fluorphenylamino)-propionsäure-isopropylester
4-(4'-Trifluormethyl-phenoxy)-penten-2-carbonsäureethylester

2-Chlor-4-ethylamino-6-isopropylamino-1,3,5-triazin
2-Chlor-4-ethylamino-6-(amino-2′-propionitril)-1,3,5-triazin
2-Chlor-4-ethylamino-6-2-methoxypropyl-2-amino-1,3,5-triazin
2-Chlor-4-ethylamino-6-butin-1-yl-2-amino-1,3,5-triazin
2-Chlor-4,6-bisethylamino-1,3,5-triazin
2-Chlor-4,6-bisisopropylamino-1,3,5-triazin
2-Chlor-4-isopropylamino-6-cyclopropylamino-1,3,5-triazin

2-Azido-4-methylamino-6-isopropylamino-1,3,5-triazin
2-Methylthio-4-ethylamino-6-isopropylamino-1,3,5-triazin
2-Methylthio-4-ethylamino-6-tert-butylamino-1,3,5-triazin
2-Methylthio-4,6-bisethylamino-1,3,5-triazin
2-Methylthio-4,6-bisisopropylamino-1,3,5-triazin
2-Methoxy-4-ethylamino-6-isopropylamino-1,3,5-triazin
2-Methoxy-4,6-bisethylamino-1,3,5-triazin
2-Methoxy-4,6-bisisopropylamino-1,3,5-triazin
4-Amino-6-tert.-butyl-3-methylthio-4,5-dihydro-1,2,4-triazin-5-on

4-Amino-6-phenyl-3-methyl-4,5-dihydro-1,2,4-triazin-5-on
4-Isobutylidenamino-6-tert.-butyl-3-methylthio-4,5-dihydro-1,2,4-triazin-5-on
1-Methyl-3-cyclohexyl-6-dimethylamino-1,3,5-triazin-2,4-dion

3-tert.-Butyl-5-chlor-6-methyluracil
3-tert.-Butyl-5-brom-6-methyluracil
3-Isopropyl-5-brom-6-methyluracil
3-sec.-Butyl-5-brom-6-methyluracil
3-(2-Tetrahydropyranyl)-5-chlor-6-methyluracil
3-(2-Tetrahydropyranyl)-5,6-trimethylenuracil
3-Cyclohexyl-5,6-trimethylenuracil

2-Methyl-4-(3′-trifluormethylphenyl)-tetrahydro-1,2,4-oxadiazin-3,5-dion
2-Methyl-4-(4′-fluorphenyl)-tetrahydro-1,2,4-oxadiazin-3,5-dion
3-Amino-1,2,4-triazol
1-Allyloxy-1-(4-bromphenyl)-2-[1′,2′,4′-triazolyl-(1′)]-ethan (Salze)
1-(4-Chlorphenoxy)-3,3-dimethyl-1-(1H-1,2,4-triazol-1-yl)-butan-2-on
N,N-Diallylchloracetamid
N-Isopropyl-2-chloracetanilid
N-(Butin-1-yl-3)-2-chloracetanilid

2-Methyl-6-ethyl-N-(propargyl)-2-chloracetanilid
2-Methyl-6-ethyl-N-(ethoxymethyl)-2-chloracetanilid
2-Methyl-6-ethyl-N-(2-methoxy-1-methylethyl)-2-chloracetanilid
2-Methyl-6-ethyl-N-(isopropoxycarbonylethyl)-2-chloracetanilid
2-Methyl-6-ethyl-N-(4-methoxypyrazol-1-yl-methyl)-2-chloracetanilid
2-Methyl-6-ethyl-N-(pyrazol-1-yl-methyl)-2-chloracetanilid
2,6-Dimethyl-N-(pyrazol-1-yl-methyl)-2-chloracetanilid
2,6-Dimethyl-N-(4-methylpyrazol-1-yl-methyl)-2-chloracetatanilid
2,6-Dimethyl-N-(1,2,4-triazol-1-yl-methyl)-2-chloracetanilid
2,6-Dimethyl-N-(3,5-dimethylpyrazol-1-yl-methyl)-2-chloracetanilid
2,6-Dimethyl-N-(1,3-dioxolan-2-yl-methyl)-2-chloracetanilid
2,6-Dimethyl-N-(2-methoxyethyl)-2-chloracetanilid
2,6-Dimethyl-N-isobutoxymethyl-2-chloracetanilid
2,6-Diethyl-N-methoxymethyl-2-chloracetanilid
2,6-Diethyl-N-(n-butoxymethyl)-2-chloracetanilid
2,6-Diethyl-N-ethoxycarbonylmethyl-2-chloracetanilid
2,3,6-Trimethyl-N-(pyrazol-1-yl-methyl)-2-chloracetanilid
2,3-Dimethyl-N-isopropyl-2-chloracetanilid
2,6-Diethyl-N-(2-n-propoxyethyl)-2-chloracetanilid

2-(2-Methyl-4-chlorphenoxy)-N-methoxy-acetamid
2-(α-Naphthoxy)-N,N-diethylpropionamid
2,2-Diphenyl-N,N-dimethylacetamid
N-Benzyl-N-isopropyl-trimethylacetamid
α-(3,4,5-Tribrompyrazol-1-yl)-N,N-dimethylpropionamid
N-(1,1-Dimethylpropinyl)-3,5-dichlorbenzamid

N-1-Naphthylphthalamidsäure
Propionsäure-3,4-dichloranilid
Cyclopropancarbonsäure-3,4-dichloranilid
Methacrylsäure-3,4-dichloranilid
2-Methylpentancarbonsäure-3,4-dichloranilid
5-Acetamido-2,4-dimethyl-trifluormethansulfonanilid
5-Acetamido-4-methyl-trifluormethansulfonanilid
N-4-Methyl-5-(trifluormethyl)-sulfonylamino-phenylacetamid
2-Propionyl-amino-4-methyl-5-chlor-thiazol
O-(Methylsulfonyl)-glykolsäure-N-ethoxymethyl-2,6-dimethylanilid
O-(Methylaminosulfonyl)-glykolsäure-N-isopropyl-anilid
O-(i-Propylaminosulfonyl)-glykolsäure-N-butin-1-yl-3-anilid
O-(Methylaminosulfonyl)-glykolsäure-hexamethylenimid
2,6-Dichlor-thiobenzamid
2,6-Dichlorbenzonitril
3,5-Dibrom-4-hydroxy-benzonitril (Salze)
3,5-Dijod-4-hydroxy-benzonitril (Salze)
3,5-Dibrom-4-hydroxy-O-2,4-dinitrophenylbenzaldoxim (Salze)
3,5-Dibrom-4-hydroxy-O-2-cyan-4-nitrophenylbenzaldoxim (Salze)
Pentachlorphenol-Natriumsalz
2,4-Dichlorphenyl-4'-nitrophenylether
2,4,6-Trichlorphenyl-4'-nitrophenylether
2-Fluor-4,6-dichlorphenyl-4'-nitrophenylether
2-Chlor-4-trifluormethylphenyl-4'-nitrophenylether

2,4'-Dinitro-4-trifluormethyl-diphenylether
2,4-Dichlorphenyl-3'-methoxy-4'-nitro-phenylether
2-Chlor-4-trifluormethylphenyl-3'-ethoxy-4'-nitro-phenylether
2-Chlor-4-trifluormethylphenyl-3'-carboxy-4'-nitro-phenylether (Salze)
2-Chlor-4-trifluormethylphenyl-3'-ethoxycarbonyl-4'-nitrophenylether
2-Chlor-4-trifluormethylphenyl-3'-(2-fluorethoxy)-4'-nitrophenylether
2-Chlor-4-trifluormethylphenyl-3'-ethoxycarbonyl-methylthio-4'-nitrophenylether
2-Chlor-4-trifluormethyl-3'-methoxycarbonyl-4'-nitrophenylether
2,4,6-Trichlorphenyl-3'-ethoxycarbonyl-methylthio-4'-nitrophenylether
2,4-Dichlorphenyl-3'-methoxycarbonyl-4'-nitro-phenylether
2,4-Dichlorphenyl-3'-carboxy-4'-nitrophenylether
2-(3,4-Dichlorphenyl)-4-methyl-1,2,4-oxadiazolidin-3,5-dion
2-(3-tert.-Butylcarbamoyloxy-phenyl)-4-methyl-1,2,4-oxadiazolidin-3,5-dion
2-(3-i-Propylcarbamoyloxy-phenyl)-4-methyl-1,2,4-oxadiazolidin-3,5-dion
2-Phenyl-3,1-benzoxazinon-(4)
(4-Bromphenyl)-3,4,5,9,10-pentaazatetracyclo-[5,4,1,$0^{2,6}$,$0^{8,11}$]-dodeca-3,9-dien
2-Ethoxy-2,3-dihydro-3,3-dimethyl-5-benzofuranyl-methansulfonat
2-Ethoxy-2,3-dihydro-3,3-dimethyl-5-benzofuranyl-dimethylaminosulfat
2-Ethoxy-2,3-dihydro-3,3-dimethyl-5-benzofuranyl-(N-methyl-N-acetyl)-aminosulfonat
3,4-Dichlor-1,2-benzisothiazol
N-4-Chlorphenyl-allylbernsteinsäureimid
2-Methyl-4,6-dinitrophenol (Salze, Ester)
2-sec.-Butyl-4,6-dinitrophenol (Salze, Ester)
2-sec.-Butyl-4,6-dinitrophenol-acetat
2-tert.-Butyl-4,6-dinitrophenol-acetat
2-tert.-Butyl-4,6-dinitrophenol (Salze)
2-tert.-Butyl-5-methyl-4,6-dinitrophenol (Salze)
2-tert.-Butyl-5-methyl-4,6-dinitrophenol-acetat

2-sec.-Amyl-4,6-dinitrophenol (Salze, Ester)
1-(α,α-Dimethylbenzyl)-3-(4-methylphenyl)-harnstoff
1-Phenyl-3-(2-methylcyclohexyl)-harnstoff
1-Phenyl-1-benzoyl-3,3-dimethyl-harnstoff

1-(4-Chlorphenyl)-1-benzoyl-3,3-dimethyl-harnstoff
1-(4-Chlorphenyl)-3,3-dimethyl-harnstoff
1-(4-Chlorphenyl)-3-methyl-3-butin-1-yl-3-harnstoff
1-(3,4-Dichlorphenyl)-3,3-dimethyl-harnstoff
1-(3,4-Dichlorphenyl)-1-benzoyl-3,3-dimethyl-harnstoff
1-(3,4-Dichlorphenyl)-3-methyl-3-n-butyl-harnstoff

1-(4-i-Propylphenyl)-3,3-dimethyl-harnstoff
1-(3-Trifluormethylphenyl)-3,3-dimethyl-harnstoff
1-(3-α,α,β,β-Tetrafluorethoxyphenyl)-3,3-dimethyl-harnstoff
1-(3-tert.-Butylcarbamoyloxy-phenyl)-3,3-dimethyl-harnstoff
1-3-Chlor-4-methylphenyl)-3,3-dimethyl-harnstoff
1-(3-Chlor-4-methoxyphenyl)-3,3-dimethyl-harnstoff
1-(3,5-Dichlor-4-methoxyphenyl)-3,3-dimethyl-harnstoff
1-[4-(4'-Chlorphenoxy)-phenyl]-3,3-dimethyl-harnstoff
1-[4-(4'-Methoxyphenoxy)-phenyl]-3,3-dimethyl-harnstoff
1-Cyclooctyl-3,3-dimethyl-harnstoff
1-(Hexahydro-4,7-methanindan-5-yl)-3,3-dimethyl-harnstoff
1-[1- oder 2-(3a,4,5,7,7a-Hexahydro)-4,7-methanoindanyl]-3,3-dimethyl-harnstoff
1-(4-Fluorphenyl)-3-carboxymethoxy-3-methyl-harnstoff
1-Phenyl-3-methyl-3-methoxy-harnstoff
1-(4-Chlorphenyl)-3-methyl-3-methoxy-harnstoff
1-(4-Bromphenyl)-3-methyl-3-methoxy-harnstoff
1-(3,4-Dichlorphenyl)-3-methyl-3-methoxy-harnstoff
1-(3-Chlor-4-bromphenyl)-3-methyl-3-methoxy-harnstoff
1-(3-Chlor-4-isopropylphenyl)-3-methyl-3-methoxy-harnstoff
1-(3-Chlor-4-methoxyphenyl)-3-methyl-3-methoxy-harnstoff
1-(3-tert.-Butylphenyl)-3-methyl-3-methoxy-harnstoff
1-(2-Benzthiazolyl)-1,3-dimethyl-harnstoff
1-(2-Benzthiazolyl)-3-methyl-harnstoff
1-(5-Trifluormethyl-1,3,4-thiadiazolyl)-1,3,4-thiadiazolyl)-1,3-dimethyl-harnstoff
1-(4-Benzyloxyphenyl)-3-methyl-3-methoxy-harnstoff
Imidazolidin-2-on-1-carbonsäure-isobutylamid

1,2-Dimethyl-3,5-diphenylpyrazolium-methylsulfat
1,2-4-Trimethyl-3,5-diphenylpyrazolium-methylsulfat
1,2-Dimethyl-4-brom-3,5-diphenylpyrazolium-methylsulfat
1,3-Dimethyl-4-(3,4-dichlorbenzoyl)-5-[(4-methylphenyl)-sulfonyloxy]-pyrazol
1-Acetyl-3-anilino-4-methoxycarbonyl-5-methyl-pyrazol
3-Anilino-4-methoxycarbonyl-5-methyl-pyrazol
3-tert.-Butylamino-4-methoxycarbonyl-5-methyl-pyrazol
2,3,5-Trichlor-pyridinol-(4)
1-Methyl-3-phenyl-5-(3'-trifluormethylphenyl)-pyridon-(4)
1-Methyl-4-phenyl-pyridiniumchlorid
1,1-Dimethylpyridiniumchlorid
3-Phenyl-4-hydroxy-6-chlorpyridazin
1,1'-Dimethyl-4,4'-dipyridylium-di-methylsulfat
1,1'-Di-(3,5-dimethylmorpholin-carbonylmethyl)-4,4'-dipyridylium-dichlorid
1,1'-Ethylen-2,2'-dipyridylium-dibromid
3-[1(N-Ethoxyamino)-propyliden]-6-ethyl-3,4-dihydro-2H-pyran-2,4-dion
3-[1-N-Allyloxyamino)-propyliden]-6-ethyl-3,4-dihydro-2-H-pyran-2,4-dion
2-[1-(N-Allyloxyamino)-propyliden]-5,5-dimethylcyclohexan-1,3-dion (Salze)
2-[1-(N-Allyloxyamin)-butyliden]-5,5-dimethylcyclohexan-1,3-dion (Salze)
2-[1-(N-Allyloxyamino)-butyliden]-5,5-dimethyl-4-methoxycarbonyl-cyclohexan-1,3-dion (Salze)

2-Chlorphenoxyessigsäure (Salze, Ester, Amide)
4-Chlorphenoxyessigsäure (Salze, Ester, Amide)
2,4-Dichlorphenoxyessigsäure (Salze, Ester, Amide)
2,4,5-Trichlorphenoxyessigsäure (Salze, Ester, Amide)
2-Methyl-4-chlorphenoxyessigsäure (Salze, Ester, Amide)
3,5,6-Trichlor-2-pyridinyl-oxyessigsäure (Salze, Ester, Amide)-Naphthoxyessigsäuremethylester
2-[4-(5'-Brompyridyl-2-oxy)-phenoxy]-propionsäureethylester
2-[4-(5'-Iodpyridyl-2-oxy)-phenoxy]-propionsäureethylester
2-[4-(5'-Iodpyridyl-2-oxy)-phenoxy]-propionsäure-n-butylester
2-(2-Methylphenoxy)-propionsäure (Salze, Ester, Amide)
2-(4-Chlorphenoxy)-propionsäure (Salze, Ester, Amide)
2-(2,4-Dichlorphenoxy)-propionsäure (Salze, Ester, Amide)
2-(2,4,5-Trichlorphenoxy)-propionsäure (Salze, Ester, Amide)
2-(2-Methyl-4-chlorphenoxy)-propionsäure (Salze, Ester, Amide)
2-[4-(4'-Chlorphenoxymethyl)-phenoxy]-propionsäuremethylester
4-(2,4-Dichlorphenoxy)-buttersäure (Salze, Ester, Amide)

4-(2-Methyl-4-chlorphenoxy)-buttersäure (Salze, Ester, Amide)
Cyclohexyl-3-(2,4-dichlorphenoxy)-acrylat
9-Hydroxyfluoren-carbonsäure-(9) (Salze, Ester)
2,3,6-Trichlorphenyl-essigsäure (Salze, Ester)
4-Chlor-2-oxo-benzothiazolin-3-yl-essigsäure (Salze, Ester)

Gibellerinsäure (Salze)
Dinatrium-methylarsonat
Mononatriumsalz der Methylarsonsäure
N-Phosphon-methyl-glycin (Salze)
N,N-Bis-(phosphonmethyl)-glycin (Salze)
2-Chlorethanphosphonsäure-2-chlorethylester
Ammonium-ethyl-carbamoyl-phosphonat
Di-n-butyl-1-n-butylamino-cyclohexyl-phosphonat
Trithiobutylphosphit
O,O-Diisopropyl-5-(2-benzosulfonylamino-ethyl)-phosphordithionat

2,3-Dihydro-5,6-dimethyl-1,4-dithiin-1,1,4,4-tetraoxid
5-tert.-Butyl-3-(2,4-dichlor-5-isopropoxyphenyl)-1,3,4-oxadiazolon-(2)
4,5-Dichlor-2-trifluormethyl-benzimidazol (Salze)
1,2,3,6-Tetrahydropyridazin-3,6-dion (Salze)
Bernsteinsäure-mono-N-dimethylhydrazid (Salze)
(2-Chlorethyl)-trimethyl-ammoniumchlorid
(2-Methyl-4-phenylsulfonyl)-trifluormethansulfonanilid
1,1-Dimethyl-4,6-diisopropyl-5-indanylethylketon
2-[1-(2,5-Dimethylphenyl)-ethylsulfonyl]-pyridin-N-oxid
2-(3'-Trifluormethyl-phenyl)-4H-3,1-benzoxazin-4-on
2-(2-Thienyl)-4H-3,1-benzoxazin-4-on
Natriumchlorat
Ammoniumrhodanid
Calciumcyanamid

2-Chlor-4-trifluormethylphenyl-3'-ethoxycarbonyl-4'-nitrophenylether
1-(4-Benzyloxyphenyl)-3-methyl-3-methoxyharnstoff
2-[1-(2,5-Dimethylphenyl)-ethylsulfonyl]-pyridin-N-oxid
1-Acetyl-3-anilino-4-methoxycarbonyl-5-methylpyrazol
3-Anilino-4-methoxycarbonyl-5-methylpyrazol
3-tert. Butylamino-4-methoxycarbonyl-5-methylpyrazol
N-Benzyl-N-isopropyl-trimethylacetamid
2-[4-(4'-Chlorphenoxymethyl)-phenoxy]-propionsäuremethylester
2-[4-(5'-Iodpyridyl-2-oxy)-phenoxy]-propionsäureethylester
2-[4-(5'-Iodpyridyl-2-oxy)-phenoxy]-propionsäure-n.-butylester
2-Chlor-4-trifluormethylphenyl-3'-(2 fluor ethoxy)-4'-nitrophenylether
2-Chlor-4-trifluormethylphenyl-3(ethoxycarbonyl)methylthio-4-nitrophenyleter
2,4,6-Trichlorphenyl-3(ethoxycarbonyl)methylthio-4-nitrophenylether
2-[1-(N-ethoxamino)-butyliden]-5-(2-ethylthiopropyl)-3-hydroxy-cyclohexen-(2)-on-(1) (Salze)
2-[1-(N-ethoxamino)-butyliden]-5-(2-phenylthiopropyl)-3-hydroxy-cyclohexen-(2)-on-(1) (Salze)

4-[4-(4'-Trifluormethyl)-phenoxy]-penten-2-carbonsäureethylester

2-Chlor-4-trifluormethyl-3'methoxycarbonyl-4'-nitrophenylether
2,4-Dichlorphenyl-3'-carboxy-4'-nitrophenylether (Salze)
4,5-Dimethoxy-2-(3-α,α,β-trifluor-β-bromethoxyphenyl)-3-(2H)-pyridazinon
2,4-Dichlorphenyl-3'-ethoxy-ethoxy-ethoxy-4'-nitrophenylether
2,3-Dihydro-3,3-dimethyl-5-benzofuranyl-ethansulfonat
N-[4-Methoxy-6-methyl-1,3,5-triazin-2-yl-aminocarbonyl]-2-chlorbenzolsulfonamid
1(3-Chlor-4-ethoxyphenyl)-3,3-dimethylharnstoff
2-Methyl-4-Chlorphenoxy-thioessigsäureethylester
2-Chlor-3,5-dijod-4-acetoxy-piridin
1(-4-[2-(4-Methylphenyl)-äthoxy]-phenyl)-3-methyl-3-methoxyharnstoff
2,6-Dimethyl-N-(pyrazol-1-yl-methylenoxymethyl)-2-chloracetanilid
2-Methyl-6-ethyl-N-(pyrazol-1-yl-methylenoxymethyl)-2-chloracetanilid
1-(α-2,4-Dichlorphenoxypropionsäure)-3-(O-methylcarbamoyl)-anilid
1-(α-2-Brom-4-chlorphenoxypropionsäure)-3-(O-methylcarbamoyl)-anilid
2-Methyl-6-ethyl-N-(pyrazol-1-yl-äthylenoxymethyl)-2-chloracetanilid

Methyl-N-dichlorfluormethylsulfenyl-(3-(N'-dichlorfluormethylsulfenyl-N'-phenylcarbamoyl-oxy)-phenyl)-carbamat

Methyl-N-dichlorfluormethylsulfenyl-(3-(N'-dichlorfluormetysulfenyl-3'-3-methylphenylcarbamoyl-oxy)-phenyl) carbamat

N-(Pyrazol-1-yl-methyl)-pyrazol-1-yl-essigsäure-2,6-dimethylanilid

N-(Pyrazol-1-yl-methyl)-1,2,4-triazol-1-yl-essigsäure-2,6-dimethylanilid

2-(3'Trifluormethylphenyl)-4H-3,1-benzoxazin-4-on 2-(2-thienyl)-4H-3,1-benzoxazin-4-on

Außerdem ist es nützlich, die neuen Verbindungen allein oder in Kombination mit anderen Herbiziden auch noch mit weiteren Pflanzenschutzmitteln gemischt gemeinsam anszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden. Es können auch nichtphytotoxische Öle und Ölkonzentrate zugesetzt werden.

Tabelle 1 — Liste der Pflanzennamen

| Botanischer Name | Abkürzung in Tabelle | Deutscher Name | Englischer Name |
|---|---|---|---|
| Abutilon theophrasti | Abuti. theoph. | Chinesischer Hanf | velvet leaf |
| Avena sativa | - | Hafer | Oats |
| Brassica napus | - | Raps | rape seed |
| Centaurea cyanus | - | Kornblume | Cornflower |
| Chenopodium album | Cheno. album | Weißer Gänsefuß | lambsquarters |
| Datura stramonium | Datura stram. | gemeiner Stechapfel | jimsonweed |
| Galium aparine | - | Klettenlabkraut | catchweed bedstraw |
| Gossypium hirsutum | - | Baumwolle | cotton |
| Ipomoea spp. | - | Prunkwindearten | morningglory |
| Lamium spp. | - | Taubnesselarten | henbit |
| Malva neglecta | Malva negle. | Weg-Malve | common mallow |
| Oryza sativa | - | Reis | rice |
| Sinapis alba | - | Weißer Senf | white mustard |
| Solanum nigrum | - | Schwarzer Nachtschatten | black nightshade |
| Triticum aestivum | Tritic. aestiv. | Weizen | wheat |
| Veronica spp. | - | Ehrenpreis | - |

**Ansprüche** (für die Vertragsstaaten : BE, CH, DE, FR, GB, IT, LI, LU, NL, SE)

1. 5-Amino-1-phenylpyrazol-4-carbonsäureester der Formel

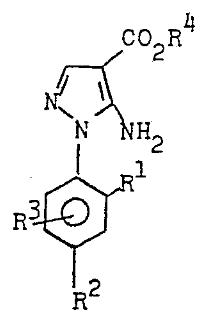

in der
$R^1$ Methyl, Trifluormethyl, Chlor oder Brom,
$R^2$ Chlor, Brom, Jod oder $C_1$-$C_3$-Alkylsulfonyl,
$R^3$ Wasserstoff, Chlor oder Brom bedeutet oder Methoxi in 5-Stellung bedeutet, wobei dann $R^1$ und $R^2$ Chlor bedeuten, oder $R^2$ Wasserstoff bedeutet, wenn $R^3$ Chlor in 3-Stellung bedeutet und
$R^4$ Methyl bedeutet.

2. Herbizid enthaltend einen 5-Amino-1-phenylpyrazol-4-carbonsäureester der Formel

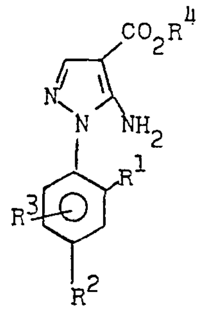

in der
$R^1$ Methyl, Trifluormethyl, Chlor oder Brom,
$R^2$ Chlor, Brom, Jod oder $C_1$-$C_3$-Alkylsulfonyl,
$R^3$ Wasserstoff, Chlor oder Brom bedeutet oder Methoxi in 5-Stellung bedeutet, wobei dann $R^1$ und $R^2$ Chlor bedeuten oder $R^2$ Wasserstoff bedeutet, wenn $R^3$ Chlor in 3-Stellung bedeutet und
$R^4$ Alkyl mit 1 bis 3 Kohlenstoffatomen bedeutet.

3. Verfahren zur Herstellung eines Herbizids, dadurch gekennzeichnet, daß man einen festen oder flüssigen Trägerstoff vermischt mit einem 5-Amino-1-phenylpyrazol-4-carbonsäureester gemäß Anspruch 2.

4. Verfahren zur Bekämpfung von unerwünschten Pflanzen dadurch gekennzeichnet, daß man die Pflanzen oder den Boden behandelt mit einem 5-Amino-1-phenylpyrazol-4-carbonsäureester gemäß Anspruch 2.

5. Verfahren zur Herstellung eines 5-Amino-1-phenyl-pyrazol-4-carbonsäureesters gemäß Anspruch 2, dadurch gekennzeichnet, daß man in einem ersten Schritt ein substituiertes Phenylhydrazin der Formel

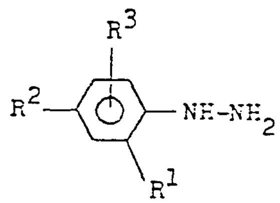

in der $R^1$, $R^2$ und $R^3$ die oben genannten Bedeutungen haben oder dessen mineralsaure Salze mit einen substituierten 2-Cyanacrylsäureester der Formel

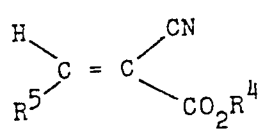

in der $R^4$ die oben angegebene Bedeutung hat und $R^5$ Alkoxi mit 1 bis 4 Kohlenstoffatomen oder N,N-Dialkylamino mit 1 bis zu 4 Kohlenstoffatomen je Alkylgruppe oder eine Hydroxigruppe bedeutet, gegebenenfalls in Gegenwart eines säurebindenden Mittels zu einem substituierten 2'-Phenylhydrazino-2-Cyanacrylsäureester der Formel

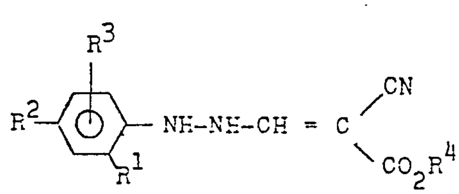

in der $R^1$, $R^2$, $R^3$ und $R^4$ die oben angegebenen Bedeutungen haben, bei Temperaturen unterhalb 70 °C

umsetzt und diese Verbindung in einem zweiten Schritt durch Erhitzen auf Temperaturen über 70 °C in einem Lösungsmittel oder Behandeln mit wäßriger Mineralsäure bei 0° bis 150 °C cyclisiert.

6. 5-Amino-1-(2,4,6-trichlorphenyl)-pyrazol-4-carbonsäuremethylester,
7. 5-Amino-1-(2,4-dichlorphenyl)-pyrazol-4-carbonsäuremethylester,
8. 5-Amino-1-(2-methyl-4-chlorphenyl)-pyrazol-4-carbonsäuremethylester,
9. 5-Amino-1-(2,4,6-tribromphenyl)-pyrazol-4-carbonsäuremethylester.

**Ansprüche** (für den Vertragsstaat AT)

1. Herbizid enthaltend einen 5-Amino-1-phenylpyrazol-4-carbonsäureester der Formel

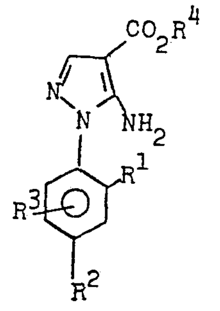

in der

$R^1$ Methyl, Trifluormethyl, Chlor oder Brom,

$R^2$ Chlor, Brom, Jod oder $C_1$-$C_3$-Alkylsulfonyl,

$R^3$ Wasserstoff, Chlor oder Brom bedeutet oder Methoxi in 5-Stellung bedeutet, wobei dann $R^1$ und $R^2$ Chlor bedeuten, oder $R^2$ Wasserstoff bedeutet, wenn $R^3$ Chlor in 3-Stellung bedeutet und

$R^4$ Alkyl mit 1 bis 3 Kohlenstoffatomen bedeutet.

2. Verfahren zur Herstellung eines Herbizids, dadurch gekennzeichnet, daß man einen festen oder flüssigen Trägerstoff vermischt mit einem 5-Amino-1-phenylpyrazol-4-carbonsäureester gemäß Anspruch 1.

3. Verfahren zur Bekämpfung von unerwünschten Pflanzen dadurch gekennzeichnet, daß man die Pflanzen oder den Boden behandelt mit einem 5-Amino-phenylpyrazol-4-carbonsäureester gemäß Anspruch 1.

4. Verfahren zur Herstellung eines 5-Amino-1-phenylpyrazol-4-carbonsäureesters gemäß Anspruch 1, dadurch gekennzeichnet, daß man in einem ersten Schritt ein substituiertes Phenylhydrazin der Formel

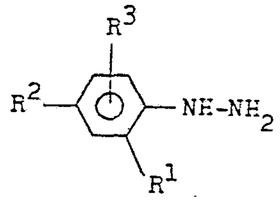

in der $R^1$, $R^2$ und $R^3$ die oben genannten Bedeutungen haben oder dessen mineralsaure Salze mit einen substituierten 2-Cyanacrylsäureester der Formel

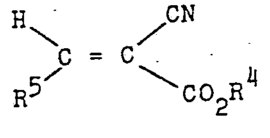

in der $R^4$ die oben angegebene Bedeutung hat und $R^5$ Alkoxi mit 1 bis 4 Kohlenstoffatomen oder N,N-Dialkylamino mit 1 bis zu 4 Kohlenstoffatomen je Alkylgruppe oder eine Hydroxigruppe bedeutet, gegebenenfalls in Gegenwart eines säurebindenden Mittels zu einem substituierten 2'-Phenylhydrazino-2-Cyanacrylsäureester der Formel

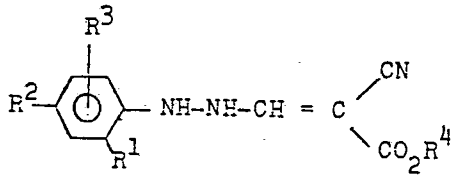

in der $R^1$, $R^2$, $R^3$ und $R^4$ die oben angegebenen Bedeutungen haben, bei Temperaturen unterhalb 70 °C umsetzt und diese Verbindung in einem zweiten Schritt durch Erhitzen auf Temperaturen über 70 °C in einem Lösungsmittel oder Behandeln mit wäßriger Mineralsäure bei 0° bis 150 °C cyclisiert.

5. Herbizid enthaltend den 5-Amino-1-(2,4,6-trichlorphenyl)-pyrazol-4-carbonsäuremethylester.

**Claims** (for the Contracting States : BE, CH, DE, FR, GB, IT, LI, LU, NL, SE)

1. A 5-amino-1-phenylpyrazole-4-carboxylic acid ester of the formula

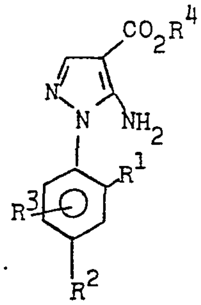

where $R^1$ is methyl, trifluoromethyl, chlorine or bromine, $R^2$ is chlorine, bromine, iodine or $C_1$-$C_3$-alkylsulfonyl, $R^3$ is hydrogen, chlorine or bromine, or is methoxy in the 5-position, in which case $R^1$ and $R^2$ are chlorine, or $R^2$ is hydrogen if $R^3$ is chlorine in the 3-position, and $R^4$ is methyl.

2. A herbicide containing a 5-amino-1-phenylpyrazole-4-carboxylic acid ester of the formula

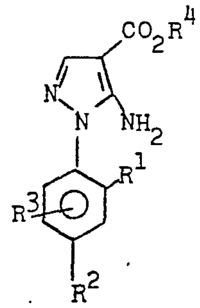

where $R^1$ is methyl, trifluoromethyl, chlorine or bromine, $R^2$ is chlorine, bromine, iodine or $C_1$-$C_3$-alkylsulfonyl, $R^3$ is hydrogen, chlorine or bromine, or is methoxy in the 5-position, in which case $R^1$ and $R^2$ are chlorine, or $R^2$ is hydrogen if $R^3$ is chlorine in the 3-position, and $R^4$ is alkyl of 1 to 3 carbon atoms.

3. A process for the manufacture of a herbicide, wherein a solid or liquid carrier is mixed with a 5-amino-1-phenylpyrazole-4-carboxylic acid ester as claimed in claim 2.

4. A process for combating unwanted plant growth, wherein the plants or the soil are treated with a 5-amino-1-phenylpyrazole-4-carboxylic acid ester as claimed in claim 2.

5. A process for the manufacture of a 5-amino-1-phenylpyrazole-4-carboxylic acid ester as claimed in claim 2, wherein, in a first step, a substituted phenylhydrazine of the formula

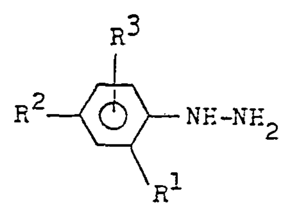

where $R^1$, $R^2$ and $R^3$ have the above meanings, or a mineral acid salt thereof, is reacted with a substituted 2-cyanoacrylic acid ester of the formula

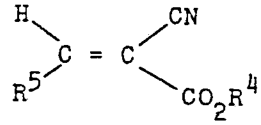

where $R^4$ has the above meaning and $R^5$ is alkoxy of 1 to 4 carbon atoms, N,N-dialkylamino, where alkyl is of 1 to 4 carbon atoms, or hydroxyl, at below 70 °C, in the presence or absence of an acid-binding agent, to give a substituted 2'-phenylhydrazino-2-cyanoacrylic acid ester of the formula

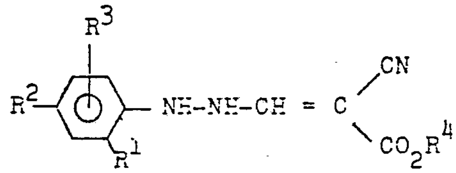

where $R^1$, $R^2$, $R^3$ and $R^4$ have the above meanings, and this compound is cyclized in a second step by heating at above 70 °C in a solvent or by treatment with aqueous mineral acid at from 0° to 150 °C.

6. Methyl 5-amino-1-(2,4,6-trichlorophenyl)-pyrazole-carboxylate.
7. Methyl 5-amino-1-(2,4-dichlorophenyl)-pyrazole-4-carboxylate.
8. Methyl 5-amino-1-(2-methyl-4-chlorophenyl)-pyrazole-4-carboxylate.
9. Methyl 5-amino-1-(2,4,6-tribromophenyl)-pyrazole-4-carboxylate.

**Claims** (for the Contracting State AT)

1. A herbicide containing a 5-amino-1-phenylpyrazole-4-carboxylic acid ester of the formula

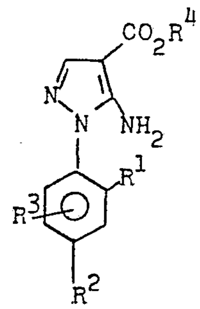

where $R^1$ is methyl, trifluoromethyl, chlorine or bromine, $R^2$ is chlorine, bromine, iodine or $C_1$-$C_3$-alkylsulfonyl, $R^3$ is hydrogen, chlorine or bromine, or is methoxy in the 5-position, in which case $R^1$ and $R^2$ are chlorine, or $R^2$ is hydrogen if $R^3$ is chlorine in the 3-position, and $R^4$ is alkyl of 1 to 3 carbon atoms.

2. A process for the manufacture of a herbicide, wherein a solid or liquid carrier is mixed with a 5-amino-1-phenylpyrazole-4-carboxylic acid ester as defined in claim 1.

3. A process for combating unwanted plant growth, wherein the plants or the soil are treated with a 5-amino-1-phenylpyrazole-4-carboxylic acid ester as defined in claim 1.

4. A process for the manufacture of a 5-amino-1-phenylpyrazole-4-carboxylic acid ester as defined in claim 1, wherein, in a first step, a substituted phenylhydrazine of the formula

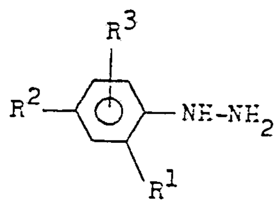

where $R^1$, $R^2$ and $R^3$ have the above meanings, or a mineral acid salt thereof, is reacted with a substituted 2-cyanoacrylic acid ester of the formula

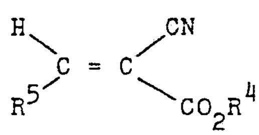

where $R^4$ has the above meaning and $R^5$ is alkoxy of 1 to 4 carbon atoms, N,N-dialkylamino, where alkyl is of 1 to 4 carbon atoms, or hydroxyl, at below 70 °C, in the presence or absence of an acid-binding agent, to give a substituted 2'-phenylhydrazino-2-cyanoacrylic acid ester of the formula

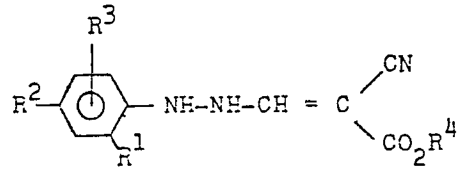

where $R^1$, $R^2$, $R^3$ and $R^4$ have the above meanings, and this compound is cyclized in a second step by heating at above 70 °C in a solvent or by treatment with aqueous mineral acid at from 0° to 150 °C.

5. A herbicide containing methyl 5-amino-1-(2,4,6-trichlorophenyl)-pyrazole-4-carboxylate.

**Revendications** (pour les Etats contractants : BE, CH, DE, FR, GB, IT, LI, LU, NL, SE)

1. Esters d'acide 5-amino-1-phénylpyrazol-4-carboxylique de formule

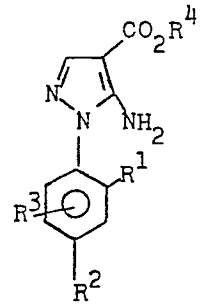

dans laquelle

$R^1$ représente méthyle, trifluorométhyle, chlore ou brome,

$R^2$ chlore, brome, iode ou alkylsulfonyle en $C_1$-$C_3$,

$R^3$ hydrogène, chlore ou brome ou méthoxy en position 5, $R^1$ et $R^2$ représentant alors chlore ou $R^2$ l'hydrogène si $R^3$ représente chlore en position 3 et

$R^4$ représente méthyle.

2. Herbicide contenant un ester d'acide 5-amino-1-phénylpyrazol-4-carboxylique de formule

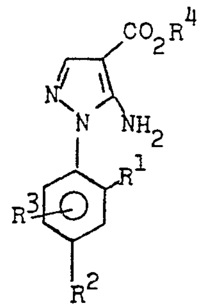

dans laquelle

$R^1$ représente méthyle, trifluorométhyle, chlore ou brome,

$R^2$ chlore, brome, iode ou alkylsulfonyle en $C_1$-$C_3$,

$R^3$ hydrogène, chlore ou brome ou méthoxy en position 5, $R^1$ et $R^2$ représentant alors chlore ou $R^2$ l'hydrogène si $R^3$ représente chlore en position 3, et

$R^4$ représente alkyle à 1 à 3 atomes de carbone.

3. Procédé de préparation d'un herbicide, caractérisé par le fait qu'on mélange un support solide ou liquide avec un ester d'acide 5-amino-1-phénylpyrazol-4-carboxylique selon la revendication 2.

4. Procédé de lutte contre les plantes indésirables, caractérisé par le fait que l'on traite les plantes ou le sol avec un ester d'acide 5-amino-1-phénylpyrazol-4-carboxylique selon la revendication 2.

5. Procédé de préparation d'un ester d'acide 5-amino-1-phénylpyrazol-4-carboxylique selon la revendication 2, caractérisé par le fait que, dans une première étape, on fait réagir, à des températures inférieures à 70 °C, une phénylhydrazine substituée de formule

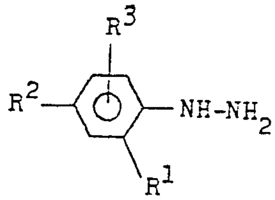

dans laquelle $R^1$, $R^2$ et $R^3$ ont les significations indiquées ci-dessus, ou ses sels d'acide minéral, avec un ester d'acide 2-cyanacrylique, substitué de formule

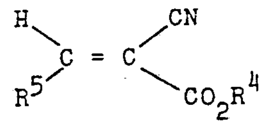

dans laquelle $R^4$ a la signification sus-indiquée et $R^5$ représente alcoxy à 1 à 4 atomes de carbone ou N,N-dialkylamino à 1 à 4 atomes de carbone pour chaque groupe alkyle ou un groupe hydroxy, éventuellement en présence d'un agent liant les acides, pour obtenir un ester d'acide 2'-phénylhydrazino-2-cyanacrylique de formule

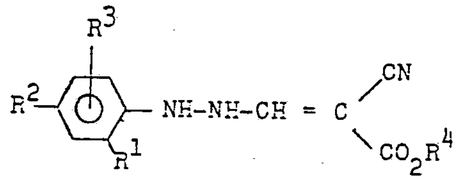

dans laquelle $R^1$, $R^2$, $R^3$ et $R^4$ ont les significations indiquées plus haut, et, dans une deuxième étape, on cyclise ce composé par chauffage à des températures supérieures à 70 °C dans un solvant ou traitement avec des acides minéraux aqueux, entre 0 et 150 °C.

6. Ester méthylique d'acide 5-amino-1-(2,4,6-trichlorophényl)-pyrazol-4-carboxylique.
7. Ester méthylique d'acide 5-amino-1-(2,4-dichlorophényl)-pyrazol-4-carboxylique.
8. Ester méthylique d'acide 5-amino-1-(2-méthyl-4-chlorophényl)-pyrazol-4-carboxylique.
9. Ester méthylique d'acide 5-amino-1-(2,4,6-tribromophényl)-pyrazol-4-carboxylique.

**Revendications** (pour l'Etat contractant AT)

1. Herbicide contenant un ester d'acide 5-amino-1-phénylpyrazol-4-carboxylique de formule

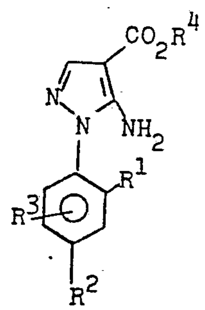

dans laquelle

$R^1$ représente méthyle, trifluorométhyle, chlore ou brome,

$R^2$ chlore, brome, iode ou alkylsulfonyle en $C_1$-$C_3$,

$R^3$ hydrogène, chlore ou brome ou méthoxy en position 5, $R^1$ et $R^2$ représentant alors chlore ou $R^2$ l'hydrogène si $R^3$ représente chlore en position 3, et

$R^4$ représente alkyle à 1 à 3 atomes de carbone.

2. Procédé de préparation d'un herbicide, caractérisé par le fait qu'on mélange un support solide ou liquide avec un ester d'acide 5-amino-1-phénylpyrazol-4-carboxylique selon la revendication 1.

3. Procédé de lutte contre les plantes indésirables, caractérisé par le fait que l'on traite les plantes ou le sol avec un ester d'acide 5-amino-1-phénylpyrazol-4-carboxylique selon la revendication 1.

4. Procédé de préparation d'un ester d'acide 5-amino-1-phénylpyrazol-4-carboxylique selon la revendication 1, caractérisé par le fait que, dans une première étape, on fait réagir, à des températures inférieures à 70 °C, une phénylhydrazine substituée de formule

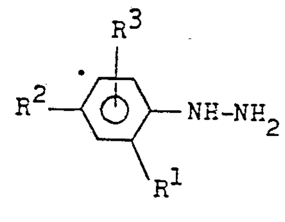

dans laquelle $R^1$, $R^2$ et $R^3$ ont les signifcations indiquées ci-dessus, ou ses sels d'acide minéral, avec un ester d'acide 2-cyanacrylique, substitué de formule

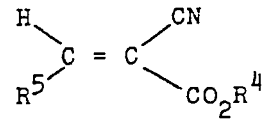

dans laquelle $R^4$ a la signification sus-indiquée et $R^5$ représente alcoxy à 1 à 4 atomes de carbone ou N,N-dialkylamino à 1 à 4 atomes de carbone pour chaque groupe alkyle ou un groupe hydroxy, éventuellement en présence d'un agent liant les acides, pour obtenir un ester d'acide 2'-phénylhydrazino-2-cyanacrylique de formule

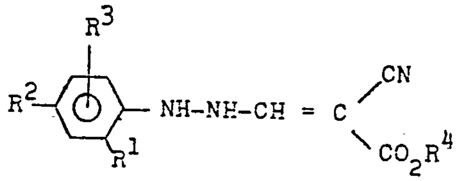

dans laquelle $R^1$, $R^2$, $R^3$ et $R^4$ ont les significations indiquées plus haut, et, dans une deuxième étape, on cyclise ce composé par chauffage à des températures supérieures à 70 °C dans un solvant ou traitement avec des acides minéraux aqueux, entre 0 et 150 °C.

5. Herbicide contenant l'ester méthylique d'acide 5-amino-1-(2,4,6-trichlorophényl)-pyrazol-4-carboxylique.